# EUROPEAN PATENT APPLICATION

(11) **EP 3 424 517 A1**
(43) Date of publication of application: **09.01.2019**
(21) Application number: 17779152.2
(22) Date of filing: 04.04.2017
(51) Int. Cl.: A61K 35/744, A61P 1/16, A61P 31/04, A61P 31/12, A61P 35/00, A61P 37/04, A61P 43/00

(54) **COMPOSITION FOR PROMOTING INTERFERON PRODUCTION AND METHOD FOR PRODUCING SAME**

(30) Priority: 04.04.2016 JP 2016075323
(71) Applicant: Kikkoman Corporation, Noda-shi, Chiba 278-8601 (JP); National Center for Global Health and Medicine, Tokyo 162-8655 (JP); National Institute of Advanced Industrial Science and Technology, Tokyo 100-8921 (JP)
(72) Inventor: YOSHIO Sachiyo, Ichikawa-shi Chiba 2728516 (JP); KANTO Tatsuya, Ichikawa-shi Chiba 272-8516 (JP); KAWASHIMA Tadaomi, Noda-shi Chiba 278-8601 (JP); IKARI Naho, Noda-shi Chiba 278-8601 (JP); TSUJI Noriko, Tsukuba-shi Ibaraki 3058566 (JP)
(74) Representative: Kramer Barske Schmidtchen Patentanwälte PartG mbB
(86) International application number: PCT/JP2017/014153
(87) International publication number: WO 2017/175774

(57) **Abstract**

An object of the present invention is to provide a composition for promoting interferon λ production, which contains an active ingredient exhibiting an effect of promoting interferon λ production with respect to BDCA3 DC, and a production method therefor. The present invention relates to (1) a composition for promoting interferon λ production, which contains a bacterial cell, a bacterial component, or a culture of lactic acid bacteria capable of being cultured under stress conditions, as an active ingredient, and (2) a method for producing a composition for promoting interferon λ production, which includes culturing lactic acid bacteria capable of being cultured under stress conditions so as to obtain a bacterial cell, a bacterial component, or a culture of the lactic acid bacteria.

## Description

### TECHNICAL FIELD

The present invention relates to a composition for promoting interferon λ production and a production method therefor.

### BACKGROUND ART

Interferon λ is one of physiologically active substances (cytokine), which belongs to the type III interferons. The production of interferon λ by dendritic cells, hepatic cells, intestinal epithelial cells, lung epithelial cells, and the like has been recognized so far. Interferon λ has an innate immunostimulatory action such as the antiviral action. Interferon λ can exhibit antiviral activity, immunostimulatory activity, anti-infective activity, anti-hepatitis B activity, anti-hepatitis C activity, antiproliferative activity, antitumor activity, anticancer activity, and the like, and thus is expected to be used for foods and drinks, therapeutic agents, prophylactic agents, improving agents, relieving agents, and the like having these activities.

Specifically, clinical trials of interferon λ have been performed with respect to a hepatitis C treatment, and it has been confirmed that interferon λ actually exhibits the antiviral action. In addition, it has been confirmed that interferon λ exhibits the antiviral action with respect to a rotavirus, an RS virus, an influenza virus, or the like, meaning that Interferon λ plays important roles in immunity of tissues such as liver, intestinal tract, and respiratory organ.

Furthermore, interferon λ is capable of supplementing a deterioration of immunity with aging. An interferon λ receptor is localized in the above-described cells in a limited manner, making interferon λ different from interferon α in which a receptor is expressed in all cells, and therefore it is possible to impart a selective physiological activity to an intake person while suppressing side effects.

A physiological activity that is expected to occur by interferon λ can be induced in a living body by increasing an in vivo expression level of interferon λ, that is, administering a substance that promotes the interferon λ production, in addition to directly administering interferon λ.

Among the cells producing interferon λ, the dendritic cells can be broadly classified into plasmacytoid dendritic cells (pDC) and myeloid dendritic cells (mDC), and mDC can be further classified into CD19-negative mDC1 and BDCA3-positive BDCA3 DC.

It has been known that subsets of these dendritic cells are different from each other in the type of cell surface molecular markers to be expressed and an expression level thereof, the type of cytokines produced, and the like (for example, refer to Non-Patent Literatures 1 and 2).

As a substance promoting the interferon λ production, for example, *Lactococcus lactis* strains JCM20101 and JCM5805 are described in Patent Literature 1 as substances capable of promoting the interferon λ production by activating pDC.

In addition, as cytokines different from interferon λ, lactic acid bacteria belonging to the genus *Tetragenococcus* have been known (for example, refer to Patent Literatures 2 and 3) as lactic acid bacteria capable of promoting production of interferon γ and interferon β.

### CITED REFERENCES

### PATENT LITERATURE

Patent Literature 1: International Publication No. 2012/091081
Patent Literature 2: JP-A-2006-028047
Patent Literature 3: Japanese Patent No. 5312322

### NON-PATENT LITERATURE

Non-Patent Literature 1: Jens Geginat et al., Frontiers in Immunology, October 2015, Vol. 6, Article 527
Non-Patent Literature 2: Bing Liu et al., Gastroenterology Research and Practice, Vol. 2015, Article ID 796461

### DISCLOSURE OF THE INVENTION

### PROBLEMS TO BE SOLVED BY THE INVENTION

According to Patent Literature 1, a production amount of interferon λ by strains JCM20101 and JCM5805 differs by one order of magnitude compared to a production amount of other interferons. From this viewpoint, the inventors of the present invention decided to investigate the effect of promoting interferon λ production by strains JCM20101 and JCM5805 once again. In the investigation, among the dendritic cells, it was decided to use BDCA3 DC which has relative large production amounts of interferon λ.

When the investigation was performed, surprisingly, strains JCM20101 and JCM5805 described in Patent Literature 1 rarely exhibited the effect of promoting interferon λ production with respect to BDCA3 DC. In addition, a substance exhibiting the effect of promoting interferon λ production with respect to BDCA3 DC is not described in Patent Literatures 2 and 3, and Non-Patent Literatures 1 and 2.

An object to be solved by the present invention is to provide a composition for promoting interferon λ production, which contains an active ingredient exhibiting the effect of promoting interferon λ production with respect to BDCA3 DC, and a production method therefor.

### MEANS FOR SOLVING THE PROBLEMS

As a result of extensive research to solve the above-described object, the inventors of the present invention have found that a bacterial cell, a bacterial component, or a culture of lactic acid bacteria capable of being cultured under stress conditions exhibits the effect of promoting interferon λ production with respect to BDCA3 DC.

The inventors of the present invention have found that, particularly surprisingly, despite the condition that strains JCM20101 and JCM5805 described in Patent Literature 1 contain double-stranded RNA, strains JCM20101 and JCM5805 do not exhibit the effect of promoting interferon λ production with respect to BDCA3 DC, whereas *Tetragenococcus halophilus* strain KK221 exhibited particularly excellent effect of promoting interferon λ production with respect to BDCA3 DC.

Based on these findings, the inventors of the present invention have succeeded in creating a composition for promoting interferon λ production, which contains, as an active ingredient, a bacterial cell, a bacterial component, or a culture of lactic acid bacteria such as strain KK221 capable of being cultured under stress conditions, and a production method therefor. The present invention has been completed based on these findings and successful examples.

That is, the present invention relates to the following aspects.
1. A composition for promoting interferon λ production, comprising:
   a bacterial cell, a bacterial component, or a culture of lactic acid bacteria capable of being cultured under stress conditions, as an active ingredient.
2. The composition for promoting interferon λ production according to above 1,
   wherein the composition for promoting interferon λ production is the composition for promoting interferon λ production with respect to BDCA3 DC.
3. The composition for promoting interferon λ production according to above 1 or 2,
   wherein the interferon λ is interferon λ3.
4. The composition for promoting interferon λ production according to any one of above 1 to 3,
   wherein the bacterial cell, the bacterial component, and the culture are the bacterial cell, the bacterial component, and the culture all of which have a nucleic acid, respectively.
5. The composition for promoting interferon λ production according to above 4,
   wherein the lactic acid bacteria capable of being cultured under the stress conditions is at least one of lactic acid bacteria comprising 15,000 ng/ml or more of double-stranded RNA per 1 × 10¹⁰ cfu of bacterial cells and lactic acid bacteria of which a ratio of double-stranded RNA in total nucleic acids is 3.5% or more per 1 × 10¹⁰ cfu of bacterial cells.
6. The composition for promoting interferon λ production according to any one of above 1 to 5,
   wherein the composition for promoting interferon λ production is an enteric composition for promoting interferon λ production.
7. Food and drink which comprise the composition for promoting interferon λ production according to any one of above 1 to 6.
8. A method for producing a composition for promoting interferon λ production, comprising:
   culturing lactic acid bacteria capable of being cultured under stress conditions so as to obtain a bacterial cell, a bacterial component, or a culture of the lactic acid bacteria.
9. A method for promoting interferon λ production, comprising:
   using a bacterial cell, a bacterial component, or a culture of lactic acid bacteria capable of being cultured under stress conditions.
10. Use of a bacterial cell, a bacterial component, or a culture of lactic acid bacteria capable of being cultured under stress conditions for manufacturing a composition for promoting interferon λ production.
11. Use of a bacterial cell, a bacterial component, or a culture of lactic acid bacteria capable of being cultured under stress conditions for promoting interferon λ production.

### EFFECT OF THE INVENTION

The composition of the present invention, and the composition obtained by the production method of the present invention have the effect of promoting interferon λ production, particularly the effect of promoting interferon λ production with respect to BDCA3 DC, and in relation to these effects, it can be expected that the composition further exhibits antiviral effects, immunostimulatory effects, anti-infective effects, resistant anti-hepatitis B effects, anti-hepatitis C effects, antiproliferative effects, antitumor effects, anticancer effects, and the like.

The active ingredient used in the composition of the present invention is an ingredient which is proven for use as additives and the like in foods and drinks. Therefore, the composition of the present invention is highly safe and is useful as antiviral agents, immunostimulants, anti-infective agents, anti-hepatitis B agents, anti-hepatitis C agents, immunostimulants for intestinal tract, immunostimulants for respiratory tract, antitumor agents, anticancer agents, and the like. It can be expected that the composition is provided in an oral or parenteral form.

### BRIEF DESCRIPTION OF DRAWINGS

[Fig. 1] Fig. 1 is a graph showing results of a test of promoting interferon λ3 production by BDCA3 DC in response to *Tetragenococcus halophilus* strain KK221, which is described in examples.
[Fig. 2] Fig. 2 is a graph showing results of a test of promoting interferon λ3 production by BDCA3 DC, pDC, and mDC1 in response to *Tetragenococcus halophilus* strain KK221, which is described in the examples.
[Fig. 3] Fig. 3 is a graph showing results of a test of promoting interferon λ3 production by BDCA3 DC in response to *Tetragenococcus halophilus* strain KK221 and bacterial cells of which the strain has been treated with RNase A, which is described in the examples.
[Fig. 4] Fig. 4 is a graph showing results of a test of promoting interferon λ3 production by BDCA3 DC according to the presence or absence of chloroquine addition, in response to *Tetragenococcus halophilus* strain KK221 and Poly IC, which is described in the examples.
[Fig. 5] Fig. 5 is a graph showing results of a test of promoting interferon λ3 production by BDCA3 DC according to the presence or absence of TRIF inhibitor addition, in response to *Tetragenococcus halophilus* strain KK221, which is described in the examples.
[Fig. 6] Fig. 6 is a graph showing results of a test of promoting interferon λ3 production by BDCA3 DC in response to *Tetragenococcus halophilus* strain KK221 and *Lactococcus lactis* strains JCM20101 and JCM5805, which is described in the examples.
[Fig. 7] Fig. 7 is a graph showing results of a test of promoting interferon α production by pDC in response to *Tetragenococcus halophilus* strain KK221 and *Lactococcus lactis* strains JCM20101 and JCM5805, which is described in the examples.
[Fig. 8] Figs. 8A and 8B are graphs showing results of each test of promoting cytokine production by BDCA3 DC in response to *Tetragenococcus halophilus* strain KK221 and Poly IC, respectively, which is described in the examples.
[Fig. 9] Fig. 9 is a graph showing results of measuring an amount of double-stranded RNA per viable cell count, in response to *Tetragenococcus halophilus* strain KK221 and *Lactococcus lactis* strains JCM20101 and JCM5805, which is described in the examples.
[Fig. 10] Fig. 10 is a graph showing results of measuring a total amount of nucleic acids per viable cell count, in response to *Tetragenococcus halophilus* strain KK221 and *Lactococcus lactis* strains JCM20101 and JCM5805, which is described in the examples.
[Fig. 11] Fig. 11 is a graph showing results of calculating a ratio of double-stranded RNA in total nucleic acids per viable cell count, in response to *Tetragenococcus halophilus* strain KK221 and *Lactococcus lactis* strains JCM20101 and JCM5805, which is described in the examples.
[Fig. 12] Fig. 12 is a graph showing results of measuring an amount of double-stranded RNA per dried bacterial cell count, in response to *Tetragenococcus halophilus* strain KK221 and *Lactococcus lactis* strains JCM20101 and JCM5805, which is described in the examples.
[Fig. 13] Fig. 13 is a graph showing results of measuring a total amount of nucleic acids per dried bacterial cell count, in response to *Tetragenococcus halophilus* strain KK221 and *Lactococcus lactis* strains JCM20101 and JCM5805, which is described in the examples.
[Fig. 14] Fig. 14 is a graph showing results of calculating a ratio of double-stranded RNA in total nucleic acids per dried bacterial cell count, in response to *Tetragenococcus halophilus* strain KK221 and *Lactococcus lactis* strains JCM20101 and JCM5805, which is described in the examples.
[Fig. 15] Fig. 15 is a graph showing results of measuring an amount of double-stranded RNA per viable cell count, in response to various lactic acid bacteria, which is described in the examples.
[Fig. 16] Fig. 16A and Fig. 16B are graphs showing results of measuring an amount of double-stranded RNA per viable cell count, with respect to 1 × 10¹⁰ cfu equivalents of liquid measures of a pasteurization-treated culture solution obtained by culturing *Lactobacillus sakei* strain K1 at 20°C, 34°C, or 38°C, which is described in the examples.
[Fig. 17] Fig. 17A and Fig. 17B are graphs showing results of measuring an amount of double-stranded RNA per viable cell count, with respect to 1 × 10¹⁰ cfu equivalents of liquid measures of a pasteurization-treated culture solution obtained by culturing *Lactobacillus sakei* strain K41 at 23°C, 29°C, or 36°C, which is described in the examples.

### EMBODIMENTS FOR CARRYING OUT THE INVENTION

Hereinafter, the present invention will be described in detail. A composition for promoting interferon λ production of the present invention (hereinafter, will simply be referred to as the composition of the present invention) has an effect of promoting interferon λ production, and contains at least a bacterial cell, a bacterial component, or a culture of lactic acid bacteria capable of being cultured under stress conditions, as an active ingredient.

The phrase "effect of promoting interferon λ production" in the present specification refers to at least one effect of promoting an expression level of genes of interferon λ, increasing a level of transcription of interferon λ proteins, or activating cells that produce interferon λ, for example.

The effect of promoting interferon λ production that the composition of the present invention has is not particularly limited as long as the effect of promoting interferon λ production is caused by providing the composition of the present invention. Examples of the effect include an effect of promoting interferon λ production by dendritic cells, preferably, an effect of promoting interferon λ production by peripheral blood dendritic cells, and more preferably, an effect of promoting interferon λ production by BDCA3 DC.

Interferon λ promoted and produced by the composition of the present invention is not particularly limited, and may be any one of interferon λ1, interferon λ2, and interferon λ3, but is preferably interferon λ3, more preferably interferon λ3 produced by BDCA3 DC.

It is expected that the composition of the present invention which has the effect of promoting interferon λ production exhibits antiviral effects, immunostimulatory effects, anti-infective effects, anti-hepatitis B effects, anti-hepatitis C effects, antiproliferative effects, antitumor effects, anticancer effects, and the like. Therefore, the composition of the present invention can adopt an aspect such as antiviral agents, immunostimulants, anti-infective agents, anti-hepatitis B agents, anti-hepatitis C agents, immunostimulants for intestinal tract, immunostimulants for respiratory tract, antitumor agents, and anticancer agents.

An anti-disease effect exhibited by the composition of the present invention means that, for example, in the case of the antiviral effects, a viral disease that a food consumer has at the present or would have in the future, or a state where the food consumer is suffering from the viral disease is suppressed, slowed down, or improved in the state.

The immunostimulatory effects exhibited by the composition of the present invention means that, for example, the present or the future immune system of the food consumer is activated, and maintained or promoted so that various diseases or abnormalities become a normal state.

As generally known, the lactic acid bacteria means a microorganism which produces a lactic acid. Examples of the lactic acid bacteria include microorganisms belonging to the genus *Tetragenococcus,* the genus *Pediococcus,* the genus *Lactobacillus,* the genus *Streptococcus,* the genus *Leuconostoc,* and the like, and from the viewpoint that the bacteria have proliferative properties, is a homo fermentative type, and generate no gas, the genus *Tetragenococcus,* the genus *Pediococcus,* and the genus *Lactobacillus* are particularly preferable.

Specific examples of the lactic acid bacteria include, but are not limited to, *Tetragenococcus halophilus* strain KK221, *Tetragenococcus halophilus* strain NBRC 12172, *Pediococcus pentosaceus* strain OS (NITE P-354), *Pediococcus pentosaceus* strain NRIC 1915, *Pediococcus pentosaceus* strain NRIC 0099, *Pediococcus pentosaceus* strain NRIC 0122, *Lactobacillus plantarum* strain NRIC 1930, *Lactobacillus plantarum* strain NRIC 1067, *Lactobacillus delbrueckii* subsp. *bulgaricus* strain NRIC 1688, *Lactobacillus delbrueckii* subsp. *lactis* strain NRIC 1683, *Lactobacillus brevis* strain NRIC 1713, *Lactobacillus pentosus* strain NRIC 0391, *Lactobacillus pentosus* strain NRIC 0396, *Lactobacillus pentosus* strain NRIC 1836, *Lactobacillus casei* subsp. *casei* strain NRIC 0644, *Lactobacillus paracasei* subsp. *paracasei* strain NRIC 1936, *Streptococcus thermophilus* strain NRIC 0256, *Leuconostoc mesenteroides* subsp. *mesenteroides* strain NRIC 1982, *Leuconostoc pseudomesenteroides* strain ATCC 12291, *Leuconostoc lactis* strain NRIC 1582, and the like.

The lactic acid bacteria capable of being cultured under the stress conditions are preferably lactic acid bacteria having large amounts of double-stranded RNA, and are more preferably at least one of lactic acid bacteria containing 15,000 ng/ml or more of double-stranded RNA per 1 × 10¹⁰ cfu of bacterial cells and lactic acid bacteria of which a ratio of double-stranded RNA in total nucleic acids is 3.5% and more per 1 × 10¹⁰ cfu of bacterial cells, among the above-described lactic acid bacteria and the like.

In a case where lactic acid bacteria are viable bacteria, the lactic acid bacteria are particularly preferably the lactic acid bacteria containing 15,000 ng/ml or more of double-stranded RNA per 1 × 10¹⁰ cfu of bacterial cells and the lactic acid bacteria of which the ratio of double-stranded RNA in the total nucleic acids is 3.5% or more per 1 × 10¹⁰ cfu of bacterial cells. In addition, in the case where lactic acid bacteria are viable bacteria, the lactic acid bacteria of which the ratio of double-stranded RNA in the total nucleic acids is 3.5% or more per 1 × 10¹⁰ cfu of bacterial cells are particularly preferable.

An upper limit of an amount of double-stranded RNA per 1 × 10¹⁰ cfu of bacterial cells of the lactic acid bacteria is not particularly limited, but is generally 50,000 ng/ml or less. In addition, an upper limit of the ratio of double-stranded RNA in the total nucleic acids per 1 × 10¹⁰ cfu of bacterial cells of the lactic acid bacteria is not particularly limited, but is generally 20% or less.

Specific aspects of the lactic acid bacteria capable of being cultured under the stress conditions include, for example, lactic acid bacteria of the genus *Tetragenococcus,* lactic acid bacteria of the genus *Pediococcus,* lactic acid bacteria of the genus *Lactobacillus,* and the like. Preferable aspects include *Tetragenococcus halophilus, Pediococcus pentosaceus, Lactobacillus sakei,* and the like, but are not limited to these examples.

As the lactic acid bacteria capable of being cultured under the stress conditions, from the viewpoint that the bacteria have the proliferative properties, is the homo fermentative type, and generate no gas, *Lactobacillus plantarum* strain NRIC 1067, *Lactobacillus delbrueckii* subsp. *lactis* strain NRIC 1683, *Lactobacillus pentosus* strain NRIC 1836, and *Streptococcus thermophilus* strain NRIC 0256 are preferable, *Pediococcus pentosaceus* strain NRIC 0099, Lactobacillus paracasei subsp. *paracasei* strain NRIC 1936, and *Lactobacillus brevis* strain NRIC 1713 are more preferable, and *Tetragenococcus halophilus* strain KK221 and *Pediococcus pentosaceus* strain OS (NITE P-354) are even more preferable.

A method for obtaining the lactic acid bacteria is not particularly limited, and examples of the method include a method of using lactic acid bacteria that are commercially available or deposited, a method of separating lactic acid bacteria from lactic acid bacteria-containing substances such as soy sauce *moromi,* pickled products, or commercially available lactic acid bacteria beverages, and using the separated bacteria.

The lactic acid bacteria contained as the active ingredient in the composition of the present invention are not particularly limited as long as they are the lactic acid bacteria capable of being cultured under the stress conditions, among the above-described lactic acid bacteria. The term "stress conditions" refers to conditions such as high salt concentration, high temperature or low temperature, oligotrophic conditions, eutrophic conditions, low pH, high pH, and the like, which are the conditions deviated from general conditions for culturing the lactic acid bacteria, and preferably refers to conditions in which a doubling time becomes longer than that of a case where the lactic acid bacteria is cultured under a condition of a suitable doubling time.

Specific examples of culturing under the stress conditions of the high salt concentration include culturing carried out by using a medium containing a salt concentration of preferably 0.5% to 30% (w/v), and more preferably 5% to 15% (w/v). In a medium in which the salt concentration is lower than 0.5% (w/v) or higher than 30% (w/v), growth of the lactic acid bacteria may be extremely slow in some cases.

In addition, examples of culturing under the stress conditions of the high temperature or low temperature include culturing carried out at an optimum temperature of ±5°C to 20°C which are suitable for the growth of the lactic acid bacteria, and preferably at the optimum temperature of ±10°C. Specific examples of the culturing include culturing carried out at 35°C to 45°C or 15°C to 25°C in a case where the optimum temperature for lactic acid bacteria to be used is 30°C, culturing carried out at 40°C to 50°C or 20°C to 30°C in a case where the optimum temperature for the lactic acid bacteria to be used is 35°C, or the like.

The lactic acid bacteria which have been cultured under the stress conditions are capable of producing more of bacterial components such as double-stranded RNA, compared to a case in which culturing is carried out under conditions with no stress.

In regard to the lactic acid bacteria capable of being cultured under the stress conditions, one kind thereof can be used or a combination of two or more kinds thereof can be used. The lactic acid bacteria contained as the active ingredient in the composition of the present invention may be lactic acid bacteria cultured under the stress conditions, may be lactic acid bacteria cultured under normal conditions, or may be any lactic acid bacteria cultured under either the stress conditions or the normal conditions. For example, one kind of two or more kinds of the lactic acid bacteria cultured under the stress conditions may be combined with one kind of two or more kinds of the lactic acid bacteria cultured under the normal conditions so as to be contained in the composition of the present invention.

The active ingredient in the composition of the present invention may be the bacterial component of the corresponding lactic acid bacteria, and the culture of the corresponding lactic acid bacteria, in addition to the lactic acid bacteria themselves capable of being cultured under the stress conditions, that is, the bacterial cell of the corresponding lactic acid bacteria. Examples of the bacterial cell of the lactic acid bacteria include a bacterial cell obtained by removing a medium from a culture of lactic acid bacteria by using a generally known solid-liquid separation means such as centrifugation.

Examples of the bacterial component of the lactic acid bacteria include a purified or unpurified component which is present within the bacterial cell of the lactic acid bacteria, or which is secreted outside the bacterial cell, and specific examples of the bacterial component include single-stranded RNA and double-stranded RNA, and single-stranded DNA and double-stranded DNA.

Examples of the culture of the lactic acid bacteria include a culture solution itself obtained by culturing the lactic acid bacteria. The active ingredient in the composition of the present invention is not particularly limited as long as it is the bacterial cell, the bacterial component, and the culture of the lactic acid bacteria capable of being cultured under the stress conditions, and for example, the bacterial cell, the bacterial component, and the culture containing a nucleic acid are preferable. As the nucleic acid, RNA is preferable, and double-stranded RNA is more preferable.

In regard to the bacterial cell, the bacterial component, and the culture of the lactic acid bacteria capable of being cultured under the stress conditions, any one of the bacterial cell, the bacterial component, and the culture can be used alone, or a combination of two or more kinds thereof can be used. Specifically, for example, from the bacterial cell, the bacterial component, and the culture of the corresponding lactic acid bacteria, RNA, preferably double-stranded RNA may be isolated to be used.

That is, the present invention also relates to the composition for promoting interferon λ production, the composition containing double-stranded RNA isolated from the bacterial cell, the bacterial component, and the culture of the lactic acid bacteria capable of being cultured under the stress conditions, as the active ingredient. Furthermore, the present invention also relates to the method for producing the composition for promoting interferon λ production, the method including obtaining the bacterial cell, the bacterial component, and the culture of the lactic acid bacteria capable of being cultured under the stress conditions, and isolating double-stranded RNA from the bacterial cell, the bacterial component, and the culture of the corresponding lactic acid bacteria.

The method for isolating RNA from the bacterial cell, the bacterial component, and the culture of the lactic acid bacteria is not particularly limited, and examples of a method for isolating double-stranded RNA from the bacterial cell of the lactic acid bacteria include the method described in Patent Literature 3.

A method for obtaining the bacterial cell, the bacterial component, or the culture of the lactic acid bacteria containing double-stranded RNA is not particularly limited. Specific examples of the method include a method in which an MRS medium (Becton, Dickinson and Company) containing 5% to 10% (w/v) salt is inoculated with lactic acid bacteria belonging to the genus *Tetragenococcus,* or a normal MRS medium is inoculated with lactic acid bacteria belonging to the genus *Pediococcus,* the genus *Lactobacillus,* the genus *Lactococcus,* the genus *Streptococcus,* and the genus *Leuconostoc,* followed by culturing at 20°C to 35°C for 24 to 72 hours, thereby obtaining the bacterial cell, the bacterial component, and the culture of the lactic acid bacteria containing double-stranded RNA.

Specific examples of the method for isolating double-stranded RNA from the bacterial cell, the bacterial component, and the culture of the lactic acid bacteria include the following method.

That is, the bacterial cell, the bacterial component, and the culture of the lactic acid bacteria containing double-stranded RNA are subjected to a heat and pasteurization treatment at 90°C to 100°C for 5 to 20 minutes, and therefore a heat and pasteurization-treated liquid is obtained. Subsequently, the heat and pasteurization-treated liquid is subjected to the solid-liquid separation means such as centrifugation, and solid contents are recovered. Subsequently, the solid contents are washed and suspended in a buffer solution to obtain a suspension. Subsequently, lysozyme is added to the suspension and warmed at 35°C to 40°C to obtain a lysozyme-treated solution.

Subsequently, SDS and Proteinase K are added to the lysozyme-treated solution and warmed at 35°C to 40°C to obtain a proteinase-treated solution. Subsequently, the proteinase-treated solution was subjected to a phenol-chloroform-isoamyl alcohol treatment, and therefore a crude nucleic acid extract is obtained as a supernatant by the solid-liquid separation means such as centrifugation. The crude nucleic acid extract is a mixture of nucleic acids which contains DNA, single-stranded RNA, double-stranded RNA, and the like.

Subsequently, by subjecting the crude nucleic acid extract to cellulose column chromatography, purified double-stranded RNA can be obtained. The cellulose column chromatography and the subsequent advanced purification means can be carried out according to the description of Patent Literature 3.

The bacterial cell, the bacterial component, and the culture of the lactic acid bacteria are proven natural products and derived products thereof, which have been ingested by humans for a long period of time, and thus are highly safe. Therefore, the composition of the present invention has high practicality, and can be applied in various applications as it is or through processing, in an oral or parenteral form.

The composition of the present invention can be mixed with, for example, other components and used, in accordance of a purpose. As described above, the composition of the present invention can be mixed with other various components in addition to the bacterial cell, the bacterial component, or the culture of the lactic acid bacteria capable of being cultured under the stress conditions, as long as the object of the present invention can be achieved by the composition.

In the composition of the present invention, for example, additives used for general food processing such as a sugar sweetener, a stabilizer, an emulsifier, a starch, a processed product of starch, a hydrolyzed product of starch, a salt, a flavoring agent, a coloring agent, an acidulant, a flavor ingredient, a nutrient, a fruit juice, animal and vegetable food ingredients such as an egg, an excipient, a bulking agent, a binder, a thickener, and a perfume oil can be further contained. An amount used of the additive is not particularly limited as long as the solution of the object of the present invention is not hindered by the amount, and the amount can be set appropriately.

The composition of the present invention is not particularly limited as long as it is in a form generally used, and for example, the composition can adopt various forms such as solid form, liquid form, gel form, suspension form, cream form, sheet form, stick form, powder form, granule form, granule form, tablet form, rod form, plate form, block form, paste form, capsule form, and caplet form.

In a case where the composition of the present invention is, for example, a composition for oral ingestion is preferably an enteric composition capable of delivering the bacterial cell, the bacterial component, and the culture of the lactic acid bacteria, and double-stranded RNA isolated therefrom to the small intestine via esophagus and stomach. The enteric composition is not particularly limited as long as it is in a form that does not dissolve by gastric acids but dissolves in the small intestine, and examples of the enteric composition include a composition in a form of an acid-resistant microcapsule or a liposome.

An amount of the bacterial cell, the bacterial component, or the culture of the lactic acid bacteria capable of being cultured under the stress conditions, which are contained in the composition of the present invention is not particularly limited as long as it is an amount by which the effect of promoting interferon λ production can be recognized. In the case of the composition for oral ingestion, the amount thereof is, for example, preferably 0.0001% by mass or more, and more preferably 0.001 % by mass or more with respect to an entire composition. An upper limit of the amount is not particularly limited and is generally 50% by mass or less. In the case of a composition for parenteral ingestion, the amount thereof is, for example, preferably 0.00001% by mass or more, more preferably 0.0001% by mass or more with respect to an entire composition. An upper limit of the amount is not particularly limited and is generally 10% by mass or less.

An ingestion dose of the composition of the present invention is not particularly limited, and may be appropriately set in accordance with a symptom and a physique of an intake person. For example, an ingestion dose of the composition containing the lactic acid bacteria capable of being cultured under the stress conditions as the active ingredient, is generally 1 to 1000 mg/60 kg of body weight/day.

The composition of the present invention can be used alone as it is, or can be used by being added to foods and drinks, medicines, and the like. That is, another aspect of the present invention include food and drink containing the composition for promoting interferon λ production of the present invention. Examples of the aspect include functional foods and drinks, foods and drinks for specified health use, nutritional functional foods and drinks, health functional foods and drinks, foods and drinks for a special purpose, nutritional supplement foods and drinks, health supplement foods and drinks, supplements, beauty foods and drinks, cosmetics, medicines, quasi-drugs, animal feed, and the like, and raw materials for producing these products.

A production method of the present invention includes at least culturing the lactic acid bacteria capable of being cultured under the stress conditions so as to obtain the bacterial cell, the bacterial component, or the culture of the corresponding lactic acid bacteria. As long as the component as the active ingredient, which exhibits the effect of promoting interferon λ production can be obtained, conditions for culturing the lactic acid bacteria, and a method for collecting the bacterial cell, the bacterial component, or the culture which are the active ingredients are not particularly limited.

Examples of one aspect of the production method of the present invention include a method in which an MRS medium with a high salt concentration or a general salt concentration is inoculated with the lactic acid bacteria, the corresponding lactic acid bacteria are cultured at the optimum temperature of ±5°C to 20°C for 12 to 72 hours, and therefore a culture is obtained, subsequently, the medium is removed from the obtained culture by using the generally known solid-liquid separation means such as ultrafiltration membrane or centrifugation, and bacterial cells are recovered, and subsequently, the obtained bacterial cells are washed with water, a saline solution, or the like, and therefore the bacterial cells of the lactic acid bacteria are obtained.

The bacterial cells obtained as above, a bacterial cell suspension in which the corresponding bacterial cells are suspended in water, a saline solution, or the like, a dry powder obtained by subjecting the corresponding bacterial cells to a drying treatment, or the like can be used as the active ingredient of the composition of the present invention. A method of the drying treatment is not particularly limited, and examples of the method include natural drying, air drying, spray drying, freeze drying, and the like.

In the production method of the present invention, various steps or operations can be added before or after the above-described steps, or during the steps, as long as the object of the present invention can be achieved.

Hereinafter, examples of the present invention will be described in detail, but the present invention is not limited to these examples, and the present invention can adopt various aspects as long as the object of the present invention can be solved.

### EXAMPLES

### [Example 1. Test of Promoting Interferon λ Production by Lactic Acid Bacteria of the Genus Tetragenococcus]

Lactic acid bacteria belonging to the genus *Tetragenococcus* were used, and a test of promoting interferon λ production was carried out.

### 1. Preparation of Lactic Acid Bacteria Suspension

*Tetragenococcus halophilus* strain KK221 (*Tetragenococcus halophilus* Th221, hereinafter will be referred to as strain KK221) was used as lactic acid bacteria. The applicant of the present application has deposited strain KK221 under the following conditions.
(1) Name of depositary institute: International Patent Organism Depositary, National Institute of Advanced Industrial Science and Technology
(2) Contact information: Central 6, 1-1, Higashi 1-chome, Tsukuba-shi, Ibaraki-ken, zip code: 305-8566 (present: Room 120, 2-5-8, Kazusa-Kamatari, Kisarazu-shi, Chiba-ken), phone number: 0438-20-5580
(3) Accession number: FERM BP-10987
(4) Identification reference: *Tetragenococcus halophilus* Th221
(5) Date of original deposite: 25 June, 2007
(6) Transfer date to a deposit under the Budapest Treaty: July 16, 2008

First, an MRS medium containing 10% (w/v) salt was inoculated with strain KK221 so that a concentration became 1 × 10⁷ cells/ml, and therefore a stock solution of lactic acid bacteria was prepared. The stock solution of lactic acid bacteria was statically cultured at 30°C for 48 to 72 hours, and then subjected to a boiling and pasteurization treatment at 95°C for 10 minutes, and therefore a pasteurization-treated culture solution was prepared. The bacterial cells obtained by centrifuging the pasteurization-treated culture solution were washed with a physiological salt solution, and then suspended in the physiological salt solution so that a concentration became 1 × 10⁹ cells/ml, and therefore a lactic acid bacteria suspension was prepared.

### 2. Test of Promoting Interferon λ Production

Activity of promoting interferon λ production by the prepared lactic acid bacteria suspension was evaluated as follows by using dendritic cells collected and prepared from the peripheral blood of a non-virus infected healthy subject.

### (1) Collection and Preparation of Peripheral Blood-Derived Dendritic Cells

The dendritic cells (DC) were isolated from 200 ml peripheral blood of the non-virus infected healthy subject by using a magnetic cell sorting system (Militeny Biotec) and a cell sorter (Becton, Dickinson and Company), as a mononuclear cell by low density gradient centrifugation. Note that, the dendritic cells were isolated in three types of phenotypic subsets of BDCA4-positive plasmacytoid DC (pDC); BDCA1-positive and CD19-negative myeloid cell type 1 (mDC1); and BDCA3-positive myeloid cell type 2 (BDCA3 DC).

The number of cells of each isolated DC was measured, and a cell stock solution was prepared using an IMDM medium (GIBCO) containing 25 mM D-glucose, 25 mM HEPES, and 1 µM sodium pyruvate so that a concentration became 5 × 10⁴ cells/ml. 200 µl of the cell stock solution per well was seeded in a 24-well tissue culture plate. Furthermore, 0 µl, 0.1 µl, 1 µl, 10 µl, and 100 µl per well of the lactic acid bacteria suspension or the IMDM medium containing the above components was added to each well (lactic acid bacteria count/DC count = 0, 10, 100, 1,000, and 10,000). The cells were cultured in a 5% CO₂ incubator at 37°C, and a culture supernatant was recovered 24 hours after the start of co-culture.

### (2) Measurement of Activity of Promoting Interferon λ Production

Using the recovered culture supernatant, interferon λ3 was measured by a chemiluminescent enzyme immunoassay method according to a method described in a document of Sugiyama, et al. [Sugiyama M, et al. Hepatol Res. 42 (11): 1089-1099, 2012].

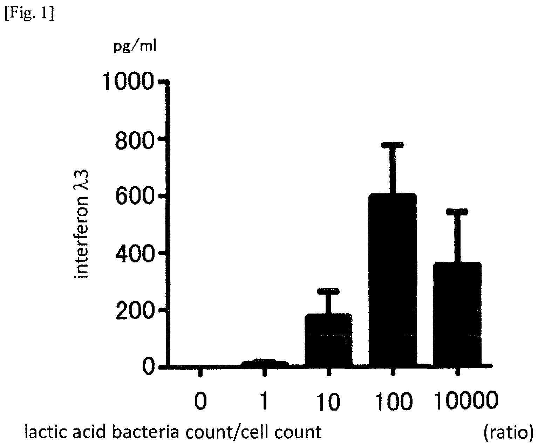

Fig. 1 shows measurement results of interferon λ3 in a case where BDCA3 DC was used as DC. As shown in Fig. 1, it was confirmed that strain KK221 promoted the interferon λ3 production by BDCA3 DC in a concentration-dependent manner at 24 hours after the start of co-culture.

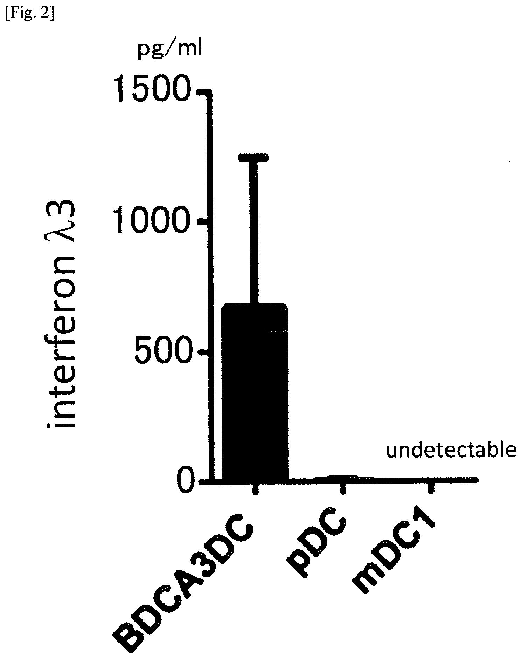

Fig. 2 shows measurement results of interferon λ3 in a case where lactic acid bacteria count/DC count was set to 100, and BDCA3 CD, pDC, and mDC1 were used as DC. As shown in Fig. 2, it was confirmed that strain KK221 promoted the interferon λ3 production by BDCA3 DC (BDCA3-positive dendritic cells) and pDC (BDCA4-positive dendritic cells) at 24 hours after the start of co-culture.

Based on the results of Figs. 1 and 2, it could be confirmed that strain KK221 had the effect of promoting interferon λ3 production in human dendritic cells.

### [Example 2. Test of Promoting Interferon λ Production by RNA Isolated from Lactic Acid Bacteria of the Genus Tetragenococcus]

### 1. RNase A Treatment

Strain KK221 was suspended so that a concentration became 5 × 10⁹ cells/ml using 10 mM Tris-HCl (pH 8.0), bovine pancreatic-derived RNase A (Sigma-Aldrich Co. LLC.) was further added thereto so that a concentration became 10 µg/ml, and therefore an RNase A-containing cell suspension was prepared.

This RNase A-containing cell suspension was subjected to an enzymatic treatment by incubating the suspension at 37°C for 2 hours, and therefore an enzyme-treated solution was obtained. In this enzymatic treatment, both single-stranded RNA and double-stranded RNA were degraded in the absence of NaCl.

The bacterial cells recovered by centrifuging the obtained enzyme-treated solution were washed twice with 10 mM Tris-HCl (pH 8.0), and then suspended in a physiological salt solution so that a concentration became 1 × 10⁹ cells/ml, and therefore an RNA-degraded lactic acid bacteria suspension was prepared.

### 2. Measurement of Activity of Promoting Interferon λ Production

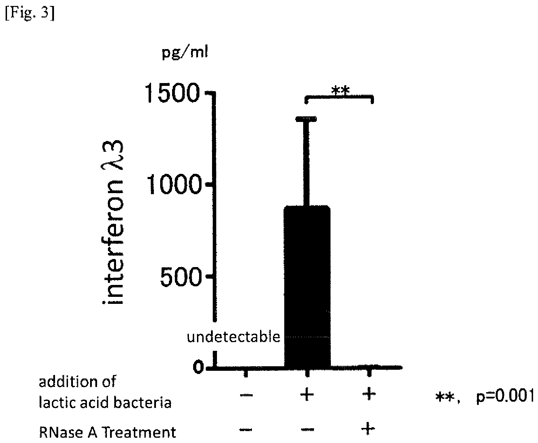

The RNA-degraded lactic acid bacteria suspension or the untreated lactic acid bacteria turbid solution prepared in Example 1, and the BDCA3 DC prepared in Example 1 were mixed at a certain ratio (BDCA3 DC count:lactic acid bacteria count = 1:100), and co-cultured. The co-cultured supernatant was recovered 24 hours after the start of culture, and a concentration of interferon λ in the supernatant was measured by the chemiluminescent enzyme immunoassay method in the same manner as in Example 1. The results are shown in Fig. 3.

As shown in Fig. 3, it was confirmed that the interferon λ production by BDCA3 DC was also promoted by using either the RNA-degraded lactic acid bacteria suspension or the untreated lactic acid bacteria turbid solution. However, it was confirmed that the activity of promoting interferon λ production deteriorated in the RNA-degraded lactic acid bacteria suspension. Based on this result, it was found that RNA in the lactic acid bacteria capable of being cultured under the stress conditions activates the dendritic cells, thereby promoting the interferon λ production. BDCA3 DC is characterized to have very high expression level of Toll-like receptor (TLR) 3. It has been known that this receptor recognizes double-stranded RNA, and it is considered that as an amount of double-stranded RNA in the bacterial cells, or a ratio of double-stranded RNA in the total nucleic acids becomes higher, the interferon λ production is strongly promoted.

### [Example 3. Test of Promoting Interferon λ Production Using TLR3 Pathway Inhibitor]

Focusing on Toll-like receptor (TLR) in BDCA3 DC, which is assumed to recognize constituents of the lactic acid bacteria, the activity of promoting interferon λ production was evaluated by using chloroquine that inhibits uptake into endosomes and inhibits the association with TLR3 present in the cytoplasm, or by using a TRIF inhibitor (product name "TRIF/TICAM1 Peptide"; Novus Biologicals) which is an adapter molecule for signal transduction through TLR3 that recognizes double-stranded RNA.

A compound Poly IC (product name "Poly IC"; InvivoGen) was used as a TLR3 ligand. Preparation of the dendritic cells, preparation of the lactic acid bacteria suspension, and measurement of the activity of promoting interferon λ production were carried out by the same method as that of Examples 1 and 2. Note that, as the lactic acid bacteria suspension, the untreated lactic acid bacteria suspension prepared in Example 1 was used. A concentration of interferon λ in the supernatant at 24 hours after the start of co-culture was measured.

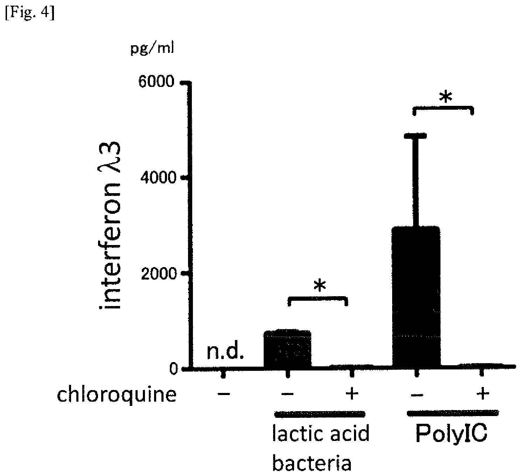

Fig. 4 shows measurement results of the activity of promoting interferon λ production according to the presence or absence of chloroquine addition for each case of addition of the lactic acid bacteria suspension and Poly IC. As shown in Fig. 4, it was confirmed that in the case where any one of the lactic acid bacteria suspension or Poly IC was added, the activity of promoting interferon λ production deteriorated according to addition of 10 µM chloroquine.

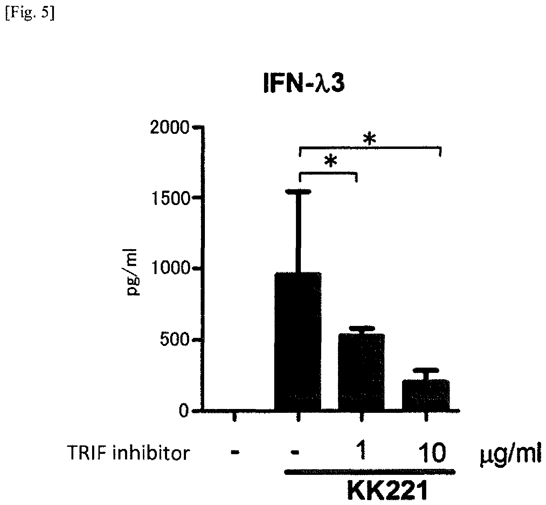

Fig. 5 shows measurement results of the activity of promoting interferon λ production by adding the lactic acid bacteria suspension and further adding 0 µg/ml, 1 µg/ml, and 10 µg/ml of the TRIF inhibitor. It was confirmed that the activity of promoting interferon λ production deteriorated by the addition of the TRIF inhibitor, depending on a concentration of the TRIF inhibitor.

Based on the results of Figs. 4 and 5, it was suggested that there is a possibility that the activity of promoting interferon λ production of BDCA3-positive dendritic cells by the lactic acid bacteria capable of being cultured under the stress conditions relates to a TRIF-mediated signaling, that is, signals from TLR3.

### [Example 4. Test of Promoting Interferon Production Using Various Lactic Acid Bacteria]

A test of promoting interferon λ production was carried out by using strain JCM20101 and strain JCM5805 (Patent Literature 1) belonging to *Lactococcus lactis* which is known to induce the interferon λ production, in addition to strain KK221.

An MRS medium not containing a salt was inoculated with JCM 20101 strain and JCM 5805 strain so that a concentration became 1 × 10⁷ cells/ml, and therefore a stock solution of lactic acid bacteria was prepared. The stock solution of lactic acid bacteria was statically cultured at 30°C for 24 hours, and then subjected to a boiling and pasteurization treatment at 95°C for 10 minutes, and therefore a pasteurization-treated culture solution was prepared. The bacterial cells obtained by centrifuging the pasteurization-treated culture solution were washed with a physiological salt solution, and then suspended in the physiological salt solution so that a concentration became 1 × 10⁹ cells/ml, and therefore a lactic acid bacteria suspension was prepared.

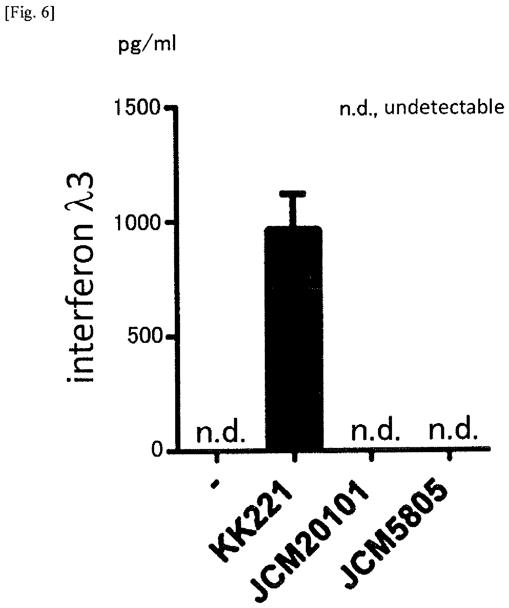

With respect to the lactic acid bacteria suspension prepared using strain JCM20101 and strain JCM5805, and the lactic acid bacteria suspension prepared using strain KK221 in Example 1, the dendritic cells were prepared as in Example 1, and under conditions in which lactic acid bacteria count/DC count = 100, the measurement of the activity of promoting interferon λ production by each lactic acid bacteria was carried out. The results are shown in Fig. 6.

As shown in Fig. 6, surprisingly, although there is the description in Patent Literature 1 that JCM20101 and strain JCM5805 promote the interferon λ production, but the effect of inducing interferon λ production with respect to BDCA3 DC was not shown in the lactic acid bacteria suspension using these strains JCM20101 and JCM5805.

Because it has been reported that the expression of interferon λ is induced by interferon α (refer to, for example, J. Viral, 2006, Vol. 80, No. 19, pp. 4501-4509), it is presumed that the induction of the interferon λ production by strain JCM20101 and strain JCM5805 described in Patent Literature 1 was generated by the result of the induction of the interferon α production by strain JCM20101 and strain JCM5805.

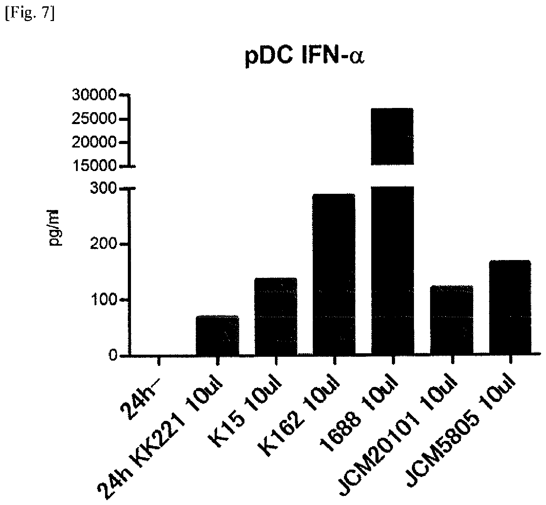

Note that, in the same manner as above, pDC and the lactic acid bacteria suspension prepared using strain KK221, strain JCM20101, and strain JCM5805 were co-cultured so that lactic acid bacteria count/DC count = 100, and the measurement of the activity of promoting interferon α production by each lactic acid bacteria was carried out. Interferon α was measured by using the recovered culture supernatant by using cytometric beads assay (CBA; Becton, Dickinson and Company) of interferon α. The results are shown in Fig. 7.

As shown in Fig. 7, it is shown that, as described in Patent Literature 1, strains JCM20101 and JCM5805 have the effect of promoting interferon α production with respect to pDC. In addition, based on the results shown in Fig. 2 that the activity of promoting interferon λ production is also shown with respect to pDC, it was suggested that the effect of promoting interferon λ production of strain JCM20101 and strain JCM5805 described in Patent Literature 1 is an effect with respect to pDC.

### [Example 5. Test of Promoting Cytokine Production by Strain KK221]

Poly IC or the lactic acid bacteria suspension prepared by using strain KK221 according to Example 1, and BDCA3 DC were prepared and co-cultured so that lactic acid bacteria count/DC count = 100, and the measurement of the activity of promoting production of various cytokines was carried out.

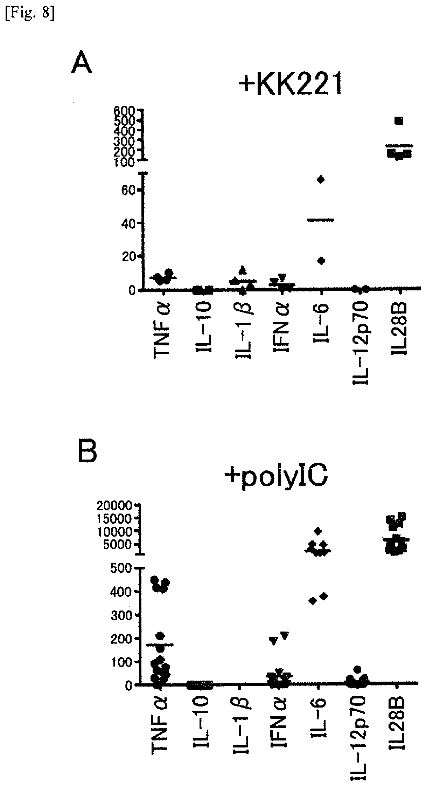

Note that each of TNFα, IL-10, IL-1β, IL-6, and IL-12p70 was measured by using cytometric beads assay (CBA; Becton, Dickinson and Company). The results are shown in Fig. 8A and Fig. 8B.

As shown in Fig. 8A and Fig. 8B, it could be confirmed that Poly IC nonspecifically promoted production of TNFα, interferon α, IL-6, and the like in addition to interferon λ (IL28B), whereas strain KK221 specifically promoted the production of interferon λ.

TNFα in which the production is induced by Poly IC is a typical inflammatory cytokine, and inflammatory reactions occur in vivo by TNFα. With respect to the above description, it was confirmed that strain KK221 can specifically promote the production of interferon λ without causing the inflammatory reactions, and thus is extremely useful for inducing the physiological effect expected by interferon λ.

### [Example 6. Evaluation of Amount and Ratio of Double-Stranded RNA per Viable Cell Count of Various Lactic Acid Bacteria]

An amount of double-stranded RNA (hereinafter also abbreviated as dsRNA) per viable cell count of strain KK221, strain JCM20101, and strain JCM5805, total amount of nucleic acids, and a ratio of double-stranded RNA in the total nucleic acids (amount of dsRNA/total amount of nucleic acids × 100) were measured as follows.

An MRS medium containing 10% (w/v) salt was inoculated with strain KK221 so that a concentration became 1 × 10⁷ cells/ml, and therefore a stock solution of lactic acid bacteria was prepared. The stock solution of lactic acid bacteria was statically cultured at 30°C for 48 hours, and therefore a culture solution of strain KK221 was obtained.

In addition, an MRS medium not containing a salt was inoculated with each of strain JCM20101 and strain JCM5805 so that a concentration became 1 × 10⁷ cells/ml, and therefore a stock solution of lactic acid bacteria was prepared. The stock solution of lactic acid bacteria was statically cultured at 30°C for 24 hours, and therefore a culture solution of strain JCM20101 and a culture solution of strain JCM5805 were obtained.

A part of the culture solution of strain KK221 was collected, serially diluted, and cultured using an MRS agar medium containing 5% (w/v) salt, and the number of colonies was counted. In addition, parts of the culture solution of strain JCM201021 and the culture solution of strain JCM5805 were collected, serially diluted, and cultured using the MRS agar medium not containing salt, and the number of colonies was counted.

A boiling and pasteurization treatment was performed on the remaining parts of the culture solution of strain KK221, the culture solution of strain JCM20101, and the culture solution of strain JCM5805 at 95°C for 15 minutes, and therefore a pasteurization-treated culture solution for each case was prepared. Based on the counted number of colonies, 1 × 10¹⁰ cfu equivalents of liquid measures of each pasteurization-treated culture solution were collected.

Each collected volume was centrifuged at 8500 × g for 15 minutes, and solid contents were recovered. Subsequently, the solid contents were then washed with a STE buffer and suspended in a STE buffer, and therefore a suspension was obtained. Then, a lysozyme (Sigma-Aldrich Co. LLC.) solution (final concentration of 5 mg/mL) suspended in the STE buffer solution was added to the suspension and warmed at 37°C for 30 minutes, and therefore a lysozyme-treated solution was obtained. Next, 10% SDS (Wako Pure Chemical Corporation) and Proteinase K (Takara Bio Inc.) were added to the lysozyme-treated solution and warmed at 37°C for 1 hour, and therefore a proteinase-treated solution was obtained.

Subsequently, the proteinase-treated solution was treated with phenolchloroform-isoamyl alcohol (Wako Pure Chemical Corporation) and centrifuged at 8500 × g for 15 minutes, and therefore a crude nucleic acid extract was obtained as a supernatant. The total amount of nucleic acids contained in the crude nucleic acid extract was measured using a microvolume spectrophotometer (product name "NanoDrop", Thermo Fisher Scientific Inc.). Double-stranded RNA from the crude nucleic acid extract was quantitatively determined by ELISA.

RNA was extracted from each collected volume, and the total amount of nucleic acids was measured using a microvolume spectrophotometer (product name "NanoDrop", Thermo Fisher Scientific Inc.). Double-stranded RNA was further extracted from the extracted RNA and quantitatively determined by ELISA.

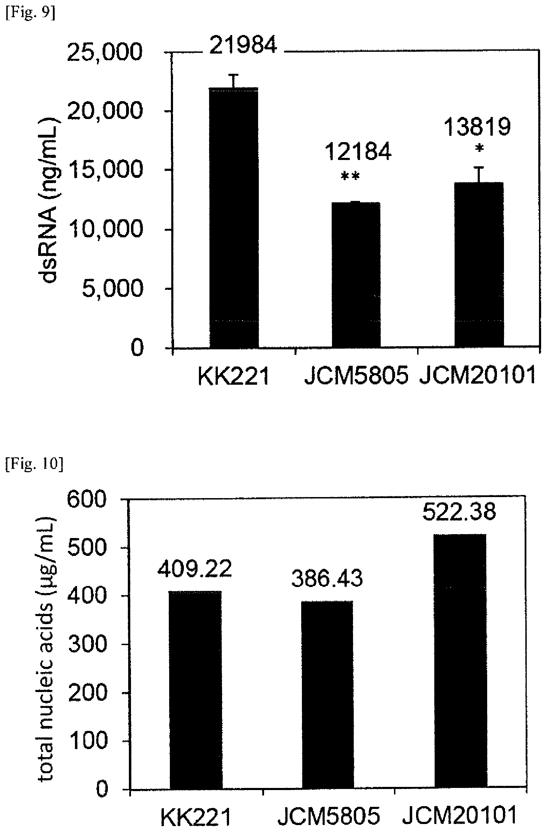

Figs. 9 to 11 respectively show the amount of double-stranded RNA, the total amount of nucleic acids, and the ratio of double-stranded RNA in the total nucleic acids which were obtained by the measurement. The examination was conducted by a comparison of two groups between strain KK221 and strain JCM20101 or strain JCM5805 (unpaired t examination). A significance level was set to 5% ("*" in the drawings) or 1% ("**") of a risk rate.

As shown in Figs. 9 to 11, the amount of double-stranded RNA of strain KK221 was larger than the amount of double-stranded RNA of strain JCM20101 and strain JCM5805. In particular, the ratio of double-stranded RNA in the total nucleic acids of strain KK221 was statistically significantly larger than that of strain JCM20101 and strain JCM5805. Based on these results, it is considered that double-stranded RNA within the lactic acid bacteria capable of being cultured under the stress conditions activates the dendritic cells to promote the interferon λ production, and as the amount of double-stranded RNA becomes larger, or the ratio of double-stranded RNA in the total nucleic acids becomes higher, the effect of promoting interferon λ production is improved.

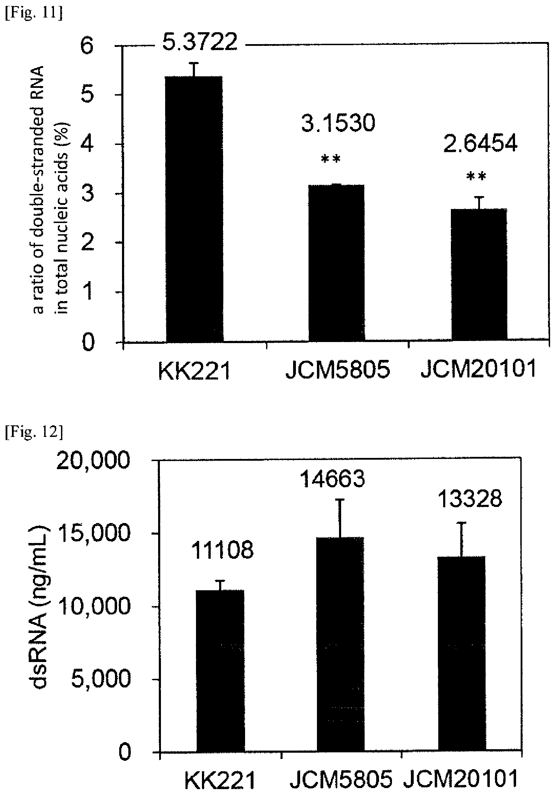

In addition, as shown in each of Fig. 9 and Fig. 11, it was found that the lactic acid bacteria in which the amount of double-stranded RNA per viable cell count is more than 12184 to 13819 ng/mL, or the ratio of double-stranded RNA in the total nucleic acids per viable cell count is more than 2.6454% to 3.1530%, is the lactic acid bacteria having the effect of promoting interferon λ production with respect to BDCA3 DC.

### [Example 7. Evaluation of Amount and Ratio of Double-Stranded RNA per Dried Bacterial Cell Count of Various Lactic Acid Bacteria]

An amount of dsRNA per dried bacterial cell count of strain KK221, strain JCM20101, and strain JCM5805, total amount of nucleic acids, and a ratio of double-stranded RNA in the total nucleic acids were measured as follows.

An MRS medium containing 10% (w/v) salt was inoculated with strain KK221 so that a concentration became 1 × 10⁷ cells/ml, and therefore a stock solution of lactic acid bacteria was prepared. The stock solution of lactic acid bacteria was statically cultured at 30°C for 48 hours, and therefore a culture solution of strain KK221 was obtained.

In addition, an MRS medium not containing a salt was inoculated with each of strain JCM20101 and strain JCM5805 so that a concentration became 1 × 10⁷ cells/ml, and therefore a stock solution of lactic acid bacteria was prepared. The stock solution of lactic acid bacteria was statically cultured at 30°C for 24 hours, and therefore a culture solution of strain JCM20101 and a culture solution of strain JCM5805 were obtained.

A boiling and pasteurization treatment was performed on the culture solution of strain KK221, the culture solution of strain JCM20101, and the culture solution of strain JCM5805 at 95°C for 15 minutes, and therefore a pasteurization-treated culture solution for each case was prepared. The bacterial cells obtained by centrifuging the pasteurization-treated culture solution were washed with a physiological salt solution, and then centrifuged again to obtain three bacterial cells, and the three bacterial cells were freeze dried.

Using 5 mg of the freeze-dried powder collected, RNA extraction, measurement of the total amount of nucleic acids, extraction of double-stranded RNA, and measurement of double-stranded RNA amount were carried out in the same manner as in Example 6. The amount of double-stranded RNA, the total amount of nucleic acids, and the ratio of double-stranded RNA in the total nucleic acids in 5 mg of each freeze-dried powder are shown in Figs. 12 to 14, respectively.

As shown in Figs. 12 to 14, the ratio of double-stranded RNA in the total nucleic acids of strain KK221 was statistically significantly larger than that of strain JCM20101 and strain JCM5805. Based on these results, it is considered that double-stranded RNA within the lactic acid bacteria capable of being cultured under the stress conditions activates the dendritic cells to promote the interferon λ production, and as the ratio of double-stranded RNA in the total nucleic acids becomes higher, the effect of promoting interferon λ production is improved.

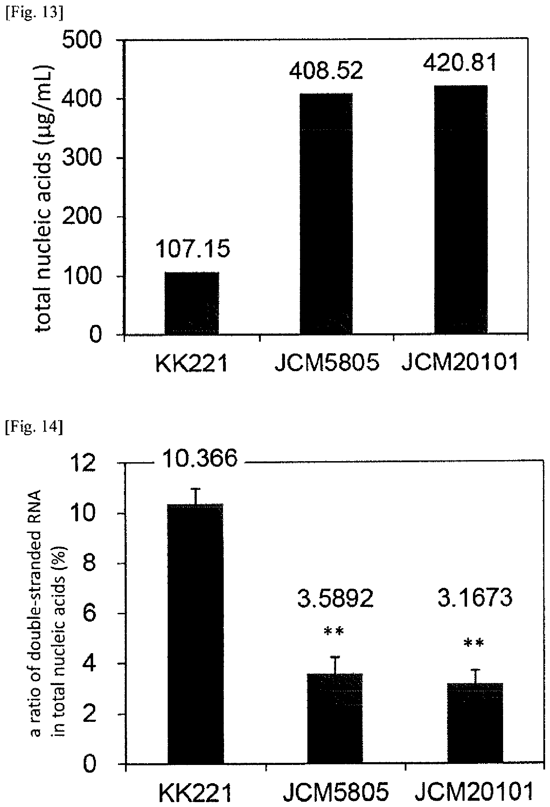

In addition, as shown in Fig. 14, it was found that the lactic acid bacteria in which the ratio of double-stranded RNA in the total nucleic acids per dried bacterial cell count is more than 3.1673% to 3.5898% is the lactic acid bacteria having the effect of promoting interferon λ production with respect to BDCA3 DC.

### [Example 8. Evaluation of Amount of Double-Stranded RNA per Viable Cell Count of Various Lactic Acid Bacteria]

The amount of double-stranded RNA per viable cell count of the various lactic acid bacteria was measured in the same manner as in Example 6, except that 5 × 10⁹ cfu equivalents of liquid measures of the pasteurization-treated culture solution were used.

As the lactic acid bacteria, *Tetragenococcus halophilus* strains KK221, K1121, and K1142; *Lactobacillus sakei* strains K1, K41, and K1185; *Lactobacillus salivarius* strains K598, K1182, and K1183; and *Lactococcus lactis* strains K436, K550, and K1118 were used.

Among these lactic acid bacteria, strain K1185 and strain K1118 have been deposited with National Institute of Technology and Evaluation, Patent Microorganisms Depositary (NITE-IPOD), and the deposit numbers are NBRC 15893 and NBRC 100933, respectively.

In addition, strain K1182 and strain K1183 were distributed from the American Type Culture Collection (ATCC), and the ATCC numbers are 13419 and 25975, respectively.

Strain K1121, strain K1142, strain K1, strain K41, strain K598, strain K436, and strain K550 are the lactic acid bacteria isolated from fermented foods and the like at Kikkoman Corporation.

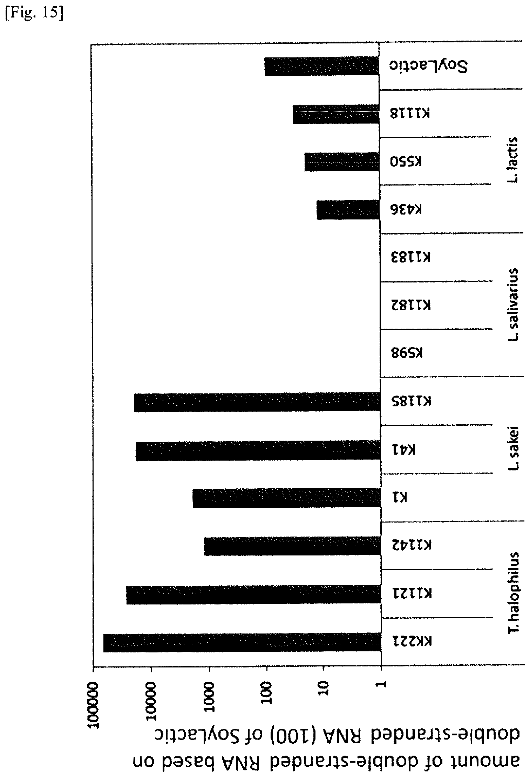

The remaining lactic acid bacteria are the strains separated and isolated by the applicant of the present invention. In addition, SoyLactic (registered trademark; Kikkoman Corporation) was used as a positive control. Fig. 15 shows measurement results of measurement values of the double-stranded RNA of each lactic acid bacteria in a case where the measurement values of double-stranded RNA of positive control was taken as 100.

As shown in Fig. 15, the three strains of *Tetragenococcus halophilus* and the three strains of *Lactobacillus sakei* had large amounts of double-stranded RNA, whereas the three strains of *Lactobacillus salivarius* and the two strains of *Lactococcus lactis* had small amounts of double-stranded RNA.

As described above, it is considered that double-stranded RNA within the lactic acid bacteria capable of being cultured under the stress conditions activates the dendritic cells through TLR3 to promote the interferon λ production, and as the amount of double-stranded RNA becomes larger, the effect of promoting interferon λ production is improved. Therefore, based on these results, it was suggested that there is a possibility that the three strains of *Tetragenococcus halophilus* and the three strains of *Lactobacillus sakei* have the effect of promoting interferon λ production with respect to BDCA3 DC.

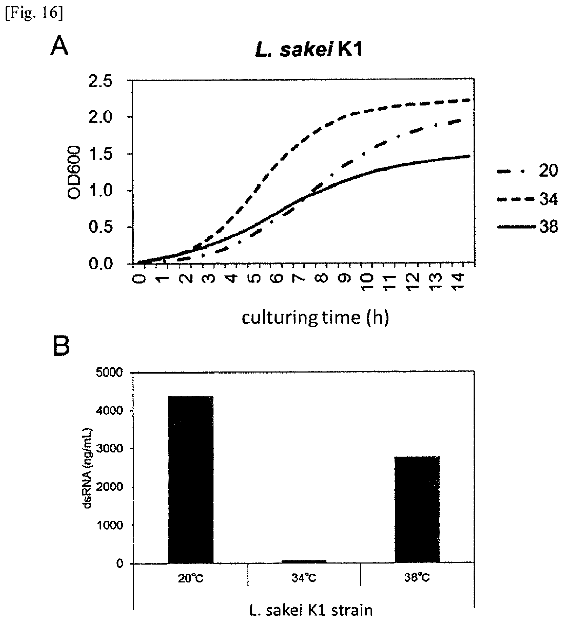

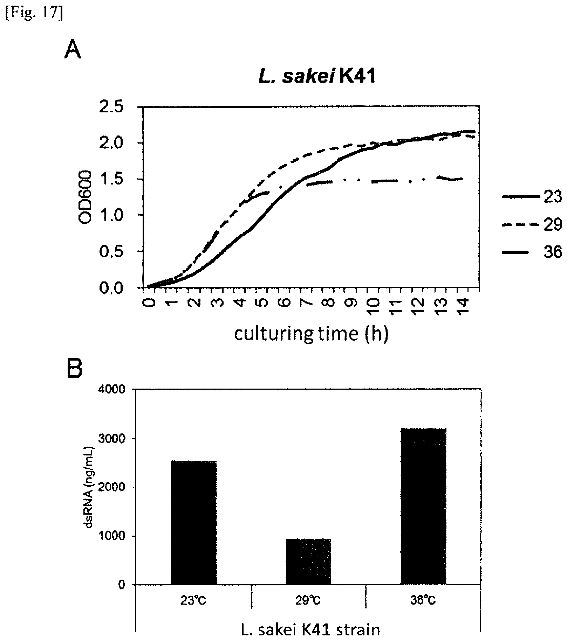

In addition, Fig. 16A, Fig. 16B, Fig. 17A and Fig. 17B respectively show results in which an amount of double-stranded RNA of *Lactobacillus sakei* strains K1 and K41 per viable cell count was measured by using 1 × 10¹⁰ cfu equivalents of liquid measures of the pasteurization-treated culture solution obtained by culturing at each temperature.

As shown in Fig. 16A, Fig. 16B, Fig. 17A and Fig. 17B, it was confirmed that the amount of double-stranded RNA of the two strains of *Lactobacillus sakei* became larger in a case of being cultured at a higher temperature (38°C or 36°C) or a lower temperature (20°C or 23°C) than the optimum temperature.

Based on these results, it was taught that the lactic acid bacteria cultured under the stress conditions have the large amounts of double stranded RNA, and thus there is a possibility that the lactic acid bacteria have the effect of promoting interferon λ production with respect to BDCA3 DC.

Although the present invention has been described in detail using the specific aspects, it is apparent to those skilled in the art that various modifications and variations are possible without departing from the spirit and scope of the present invention. Note that the present application is based on a Japanese patent application (Japanese Patent Application No. 2016-075323) filed on April 4, 2016, the entirety of which is incorporated by reference.

### INDUSTRIAL APPLICABILITY

The composition of the present invention, and the composition obtained by the production method of the present invention contain the active ingredients applicable to both oral and parenteral forms, are useful for an intake person expecting antiviral activity, immunostimulatory activity, anti-infective activity, anti-hepatitis B activity, anti-hepatitis C activity, antiproliferative activity, antitumor activity, and anticancer activity through the effect of promoting interferon λ production, and can be used as foods and drinks, medicines, quasi-drugs, cosmetics, supplements, and the like which contribute to the health and welfare of such an intake person.

## Claims

1. A composition for promoting interferon λ production, comprising:
a bacterial cell, a bacterial component, or a culture of lactic acid bacteria capable of being cultured under stress conditions, as an active ingredient.

2. The composition for promoting interferon λ production according to claim 1,
wherein the composition for promoting interferon λ production is the composition for promoting interferon λ production with respect to BDCA3 DC.

3. The composition for promoting interferon λ production according to claim 1 or 2,
wherein the interferon λ is interferon λ3.

4. The composition for promoting interferon λ production according to any one of claims 1 to 3,
wherein the bacterial cell, the bacterial component, and the culture are the bacterial cell, the bacterial component, and the culture all of which have a nucleic acid, respectively.

5. The composition for promoting interferon λ production according to claim 4,
wherein the lactic acid bacteria capable of being cultured under the stress conditions is at least one of lactic acid bacteria comprising 15,000 ng/ml or more of double-stranded RNA per 1 × 10¹⁰ cfu of bacterial cells and lactic acid bacteria of which a ratio of double-stranded RNA in total nucleic acids is 3.5% or more per 1 × 10¹⁰ cfu of bacterial cells.

6. The composition for promoting interferon λ production according to any one of claims 1 to 5,
wherein the composition for promoting interferon λ production is an enteric composition for promoting interferon λ production.

7. Food and drink which comprise the composition for promoting interferon λ production according to any one of claims 1 to 6.

8. A method for producing a composition for promoting interferon λ production, comprising:
culturing lactic acid bacteria capable of being cultured under stress conditions so as to obtain a bacterial cell, a bacterial component, or a culture of the lactic acid bacteria.

9. A method for promoting interferon λ production, comprising:
using a bacterial cell, a bacterial component, or a culture of lactic acid bacteria capable of being cultured under stress conditions.

10. Use of a bacterial cell, a bacterial component, or a culture of lactic acid bacteria capable of being cultured under stress conditions for manufacturing a composition for promoting interferon λ production.

11. Use of a bacterial cell, a bacterial component, or a culture of lactic acid bacteria capable of being cultured under stress conditions for promoting interferon λ production.
